# EUROPEAN PATENT APPLICATION

(11) **EP 4 299 748 A1**
(43) Date of publication of application: **03.01.2024**
(21) Application number: 22759951.1
(22) Date of filing: 11.02.2022
(51) Int. Cl.: C12N 15/82, C07K 14/005, C12N 15/62, A61K 39/12, A61P 31/14, A61K 47/46

(54) **METHOD FOR PRODUCING ALFALFA MOSAIC VIRUS-LIKE PARTICLES USING PLANT EXPRESSION SYSTEM AND USE THEREOF**

(30) Priority: 25.02.2021 KR 20210025902
(71) Applicant: Bioapplications Inc., Pohang-si, Gyeongsangbuk-do 37668 (KR)
(72) Inventor: PARK, Youngmin, Pohang-si, Gyeongsangbuk-do 37831 (KR); SONG, Sooji, Pohang-si, Gyeongsangbuk-do 37834 (KR); MIN, Kyungmin, Pohang-si, Gyeongsangbuk-do 37831 (KR)
(74) Representative: Handley, Matthew Edward
(86) International application number: PCT/KR2022/002106
(87) International publication number: WO 2022/182033

(57) **Abstract**

The present invention relates to a technology for constructing and producing, in the form of virus-like particles (VLPs), alfalfa mosaic virus (AMV) isolated from a plant transformed using a recombinant vector for plant expression that is targeted to the chloroplast, and provides a recombinant vector comprising a polynucleotide encoding a recombinant protein in which a chloroplast-targeting protein and an AMV capsid protein are fused. The present invention also provides a transgenic plant transformed by the recombinant vector, a method for isolating and purifying a target protein from the transgenic plant, a method for producing virus-like particles using same, and the like.

## Description

### [Technical Field]

The present invention relates to a method for producing virus-like particles (VLPs) by using a plant and the application thereof, and more specifically to a method for producing a recombinant AMV (Alfalfa mosaic virus) capsid protein by using a high-efficiency expression vector in a plant, and a method for producing the same as VLPs.

This application claims priority based on Korean Patent Application No. 10-2021-0025902, filed on February 25, 2021, and all contents disclosed in the specification and drawings of the application are incorporated herein by reference.

### [Background Art]

Virus-like particle (VLP) refers to a particle that is assembled in a shape that is similar to an actual virus through the binding of virus structural proteins. VLP does not include virus genes when assembled, and has the advantage of being a safe antigen that cannot be propagated when injected into the body. In addition, the research and development of vaccines to which VLP is applied to various pathogens are being actively conducted based on the characteristics of high immunogenicity.

Similar to viruses, VLPs can be divided into enveloped VLPs (hereinafter, "envelop VLPs") and non-enveloped VLPs (hereinafter, "non-envelop VLPs") according to the presence or absence of an envelope. In the case of envelope VLP, it is a form in which viral proteins are expressed as antigens on the outer surface while using the cell membrane of a host as an envelope, and it is produced by the process of budding outside the cell through the fusion process with the host cell membrane. Compared to non-envelope VLPs, envelope VLPs have a feature that their particle size is relatively large, and when the antigens of various pathogens are expressed on cell membranes, VLPs expressing multiple antigens can be produced at the same time. Representative envelope VLPs include influenza virus, retrovirus, hepatitis C virus VLP and the like.

In the case of non-envelope VLPs, they do not require the cell membrane of a host cell and are composed of one or several proteins among the capsid proteins of the non-enveloped virus. Non-envelope VLPs, like envelope VLPs, have the feature of being able to simultaneously express multiple antigens, but it can be said that the flexibility of simultaneously expressing antigens of other pathogens is relatively low. Representative non-envelop VLPs include hepatitis B virus, human papillomavirus, hepatitis E virus, rotavirus VLP and the like.

Currently, expression systems such as bacteria, yeast, insect cells, plant cells and higher animal (mammalian, algae, *etc*.) cells are used as expression systems for VLP production. Bacterial expression systems are most widely used to produce recombinants, but they have disadvantages in VLP production because they cannot perform post-translational modification (PTM) similar to mammalian proteins, but since the production costis low, non-envelop VLPs that can be produced only with a combination of capsid proteins are mainly used. Yeast is also widely used in the production of VLPs, but it is also used in the development of non-envelop VLPs. The development of VLPs such as hepatitis B virus and human papillomavirus has been performed by using yeast.

In the case of insect cells, except for some glycosylation patterns, the PTM process is very similar to that of mammalian cells, and thus, they can be used for both envelop and non-envelop VLP production, and generally, they have the advantage in that the production amount of proteins is high compared to higher cells. They are used in the development of VLPs against various viruses such as influenza virus and human papilloma virus.

In the case of plant cells, they are expression systems that have not been studied for a long period of time, but they have a lot of potential in terms of VLP development. Various contaminants such as viruses, cancer genes and enterotoxins that may be generated in VLP protein production can be excluded from the source, and when the demand for useful materials soars, it is absolutely advantageous compared to existing animal cell systems in terms of equipment technology and cost required for mass production, and thus, it has the advantage of being able to mass-produce at low cost in the shortest period of time. In addition, since they have a PTM similar to mammalian cells, they are characterized by being able to produce both envelop VLPs and non-envelop VLPs.

However, in spite of the above-mentioned advantages, compared to other hosts, including animal cells and microorganisms, protein production in plant cells is the biggest obstacle due to relatively unoptimized isolation and purification methods. As such, many studies are currently in progress, and attempts are being made to increase protein productivity in plant cells in various ways. For example, according to Korean Registered Patent No. 10-1848082, it has been suggested that it is possible to isolate a fusion protein including a target protein by binding a recombinant vector including cellulose binding domain 3 and proteins synthesized in a plant transformed by using the recombinant vector to cellulose, and it is possible to efficiently isolate a target protein and a cellulose binding domain by treating the fusion protein with enterokinase.

In addition, as another example of a protein isolation and purification technique that is capable of producing a target protein, according to Korean Patent Application Laid-Open No. 10-2015-0113934, when a physiologically active protein or peptide is combined with an immunoglobulin Fc fragment, it has been suggested that it has the effect of improving the solubility of physiologically active proteins or peptides compared to physiologically active proteins or peptides to which the immunoglobulin Fc fragment is not bound.

Therefore, when producing useful physiologically active substances from plants based on the related art as described above, there is an effect of being able to replace the production methods using animal cells or microorganisms, which are the existing methods. First, the period required for the production of useful physiologically active substances can be shortened, and the production cost can also be drastically reduced. Second, contaminants such as cytotoxicity that may occur in the process of isolating and purifying proteins synthesized from animal cells or microorganisms can be excluded from the source. Third, when commercialized, it can be stored for a long period of time in the form of seeds, which can reduce storage and storage costs. Fourth, since it can be transported in the form of safe seeds, it can be quickly supplied to the necessary regions and countries in case of an urgent problem. Fifth, when the demand for bioactive substances rapidly increases, it does not require a lot of technology to build production facilities and systems necessary for mass production, and thus, it can be easily mass-produced, and compared with production systems using animal cell systems, it has the advantage of dramatically lowering installation and production costs, enabling mass production in the shortest period of time, thereby enabling sufficient supply according to demand.

As described above, although various techniques for effectively synthesizing, isolating and purifying useful physiologically active substances, including medically applicable VLP proteins and industrially valuable enzymes, from plants are provided, since each protein has different unique properties, it is not desirable to apply a specific method collectively, and thus, the situation is that individual research is required.

### [Disclosure]

### [Technical Problem]

The present invention has been devised to solve the problems of the related art as described above, and in the production of recombinant AMV (Alfalfa mosaic virus) that forms virus-like particles, which are target proteins, in transgenic plants, in order to increase the expression level in the translation stage, when a Rubisco transit peptide is fused to a target protein to be targeted to the chloroplast and polyhistidine is attached for isolation and purification, it was confirmed that the protein expression level and isolation and purification efficiency are increased, and by using the same, it was confirmed that the efficiency of production of the target protein in the transgenic plant can be dramatically increased.

In addition, it was confirmed by various experimental methods that virus-like particles are formed when the composition, pH and NaCl concentration of the solution including the isolated and purified AMV as described above are appropriately adjusted. The present invention was completed by establishing a VLP platform technique that can be utilized for vaccine and therapeutic development through high-resolution tertiary structure identification of these virus-like particles.

Accordingly, an object of the present invention is to provide a recombinant vector for expressing a plant, including a polynucleotide encoding a Rubisco transit peptide including the amino acid sequence represented by SEQ ID NO: 2; and a polynucleotide encoding an Alfalfa mosaic virus (AMV) capsid protein including the amino acid sequence represented by SEQ ID NO: 4.

Another object of the present invention is to provide a transgenic plant which is transformed with the recombinant vector of the present invention.

Still another obj ect of the present invention is to provide a method for isolating and purifying the recombinant AMV capsid protein and a recombinant AMV capsid protein prepared thereby.

Still another object of the present invention is to provide a method for producing recombinant AMV virus-like particles (VLP) including the recombinant AMV capsid protein and recombinant AMV virus-like particles prepared thereby.

Still another object of the present invention is to provide a method for preparing a vaccine composition including the recombinant AMV virus-like particles according to the present invention.

Still another object of the present invention is to provide a drug delivery system including the recombinant AMV virus-like particles according to the present invention.

However, the technical problems to be achieved by the present invention are not limited to the problems mentioned above, and other problems that are not mentioned will be clearly understood by those of ordinary skill in the art to which the present invention pertains from the following description.

### [Technical Solution]

In order to achieve the objects of the present invention as described above, the present invention provides a recombinant vector for expressing a plant, including a polynucleotide encoding a Rubisco transit peptide including the amino acid sequence represented by SEQ ID NO: 2; and a polynucleotide encoding an Alfalfa mosaic virus (AMV) capsid protein including the amino acid sequence represented by SEQ ID NO: 4.

In an embodiment of the present invention, the polynucleotide encoding a Rubisco transit peptide may include the nucleotide sequence represented by SEQ ID NO: 1, and the polynucleotide encoding an AMV capsid protein may include the nucleotide sequence represented by SEQ ID NO: 3 or 10, but the present invention is not limited thereto.

In another embodiment of the present invention, the recombinant vector may further include a polynucleotide encoding a polyhistidine-tag including the amino acid sequence represented by SEQ ID NO: 6, but the presentinvention isnotlimited thereto.

In still another embodiment of the present invention, the polyhistidine-tag may be linked to the C-term end or the N-term end of the polynucleotide encoding an AMV capsid protein, but the present invention is not limited thereto.

In still another embodimentofthe present invention, in the recombinant vector, between a promoter and a terminator, (1) a polynucleotide encoding a Rubisco transit peptide, a polynucleotide encoding an AMV capsid protein and a polynucleotide encoding a polyhistidine-tag may be sequentially linked; or (2) a polynucleotide encoding a Rubisco transit peptide, a polynucleotide encoding a polyhistidine-tag and a polynucleotide encoding an AMV capsid protein may be sequentially linked, but the present invention is not limited to the linkage order.

In addition, the present invention provides a transgenic plant, which is transformed with the recombinant vector according to the present invention.

In addition, the present invention provides a method for isolating and purifying a recombinant AMV capsid protein, including the following steps:
(Step 1)transforming a plantby using the recombinant vector according to the present invention;
(Step 2) preparing a plant mixture solution by mixing the transgenic plant obtained in (Step 1) with a protein extraction buffer solution;
(Step 3) recovering a mixed solution from which plant debris is removed from the mixture solution obtained in (Step 2);
(Step 4) injecting the mixture solution obtained in (Step 3) into an agarose-filled column to adsorb a polyhistidine-tagged recombinant AMV capsid protein to the agarose;
(Step 5) washing by injecting a washing solution into the column; and
(Step 6) eluting the recombinant protein adsorbed on agarose by injecting an elution solution into the column.

In an embodiment of the present invention, the protein extraction buffer solution may satisfy at least one of the following features, but the present invention is not limited thereto:
(1) including 10 to 100 mM Tris, 100 to 1,500 mM magnesium chloride (MgCl₂), 0.01 to 1% Triton X-100 (polyethylene glycol p-(1,1,3,3-tetramethylbutyl)-phenylether), and 5 to 300 mM imidazole; or
(2) pH is 7 to 9.

In another embodiment of the present invention, the agarose may be iminodiacetic acid (Ni-IDA) agarose, but the present invention is not limited thereto.

In still another embodiment of the present invention, (Step 1) may transform a plant by using bacteria into which the recombinant vector is introduced, but the present invention is not limited thereto.

In still another embodiment of the present invention, the bacteria may be *Agrobacterium tumefaciens,* but the present invention is not limited thereto.

In still another embodiment of the present invention, the plant may be a dicotyledonous plant selected from the group consisting of Arabidopsis, soybean, tobacco, eggplant, pepper, potato, tomato, Chinese cabbage, cabbage and lettuce; or a monocotyledonous plant selected from the group consisting of rice, barley, wheat, rye, corn, sugar cane, oat and onion, but the present invention is not limited thereto.

In addition, the present invention provides a recombinant AMV capsid protein, which is eluted by the method according to the present invention.

In an embodiment of the present invention, the recombinant AMV capsid protein may satisfy at least one of the following features, but the present invention is not limited thereto:
(1) existing in the structure of a trimer; or
(2) having a molecular weight of a trimer of about 100 kDa in size-exclusion chromatography.

In addition, the present invention provides a method for producing recombinant virus-like particles (VLP), including the following steps:
(Step 1)transforming a plantby using the recombinant vector according to the present invention;
(Step 2) preparing a plant mixture solution by mixing a transgenic plant obtained in (Step 1) with a protein extraction buffer solution;
(Step 3) recovering a mixture solution from which plant debris is removed from the mixture solution obtained in (Step 2);
(Step 4) injecting the mixture solution obtained in (Step 3) into an agarose-filled column to adsorb a polyhistidine-tagged recombinant AMV capsid protein to the agarose;
(Step 5) washing by injecting a washing solution into the column;
(Step 6) injecting an elution solution into the column to elute the recombinant protein adsorbed on the agarose; and
(Step 7) preparing a protein eluted in (Step 6) into virus-like particles.

In an embodiment of the present invention, (Step 7) may prepare virus-like particles by replacing the protein extraction buffer solution including the recombinant AMV capsid protein with a buffer solution for assembling virus-like particles, but the present invention is not limited thereto.

In another embodiment of the present invention, the buffer solution for assembling virus-like particles may satisfy at least one of the following features, but the present invention is not limited thereto:
(1) including 20 to 100 mM sodium pyrophosphate and 100 to 1,000 mM sodium chloride (NaCl); or
(2) pH is 6.9 to 7.5.

In still another embodiment of the present invention, the method may further include the step of performing size exclusion chromatography to purify self-assembled recombinantAMV virus-like particles, but the present invention is not limited thereto.

In addition, the present invention provides recombinant AMV virus-like particles that are produced by the method for producing virus-like particles according to the present invention.

In an embodiment of the present invention, the recombinant AMV virus-like particles may satisfy at least one of the following features, but the present invention is not limited thereto:
(1) showing a molecular weight of about 2,000 kDa or more in size-exclusion chromatography;
(2) having a diameter of 10 nm or more to 40 nm or less when observed with a transmission electron microscope after staining by negative staining; or
(3) when observed with a cryogenic transmission electron microscope and reconstructing a 3-dimensional structure, the recombinant AMV virus-like particles are spherical VLPs (3x20) in which a total of 20 trimeric complexes are arranged in a regular arrangement.

In addition, the present invention provides a method for preparing a vaccine composition including recombinantAMV virus-like particles, including the following steps:
(Step 1)transforming a plantby using the recombinant vector according to the present invention;
(Step 2) isolating and purifying a recombinant AMV capsid protein from a transgenic plant obtained in (Step 1);
(Step 3) producing virus-like particles from the recombinant AMV capsid protein obtained in (Step 2);
(Step 4) identifying the 3D structure of the virus-like particles obtained in (Step 3) by using a Cryo-electron microscope; and
(Step 5) preparing a vaccine composition including the virus-like particles.

In an embodiment of the present invention, the method for preparing a vaccine composition may further include the step of adding an adjuvant, but the present invention is not limited thereto.

In another embodiment of the present invention, the method for preparing a vaccine composition may satisfy one of the following features, but the present invention is not limited thereto:
(1) the recombinant vector of (Step 1) further includes a polynucleotide encoding an antigen protein, and the polynucleotide encoding the antigen protein is linked to the N-term end of a polynucleotide encoding an AMV capsid protein; or
(2) (Step 5) includes the step of conjugating an antigen protein to a surface of the virus-like particles.

In addition, the present invention provides a vaccine composition, which is prepared by the method for preparing a vaccine composition.

That is, the present invention provides a vaccine composition including the AMV virus-like particles and an antigen as active ingredients.

In addition, the present invention provides the use of the AMV virus-like particles for vaccine production.

In addition, the present invention provides the use of antigen presentation or delivery of the AMV virus-like particles.

In addition, the present invention provides a use for preventing or treating a disease, including the step of administering a vaccine composition including theAMV virus-like particles and an antigen as active ingredients to a subject in need thereof.

In addition, the present invention provides a method for preparing a drug delivery system including recombinant AMV virus-like particles, including the following steps:
(Step 1)transforming a plantby using the recombinant vector according to the present invention;
(Step 2) isolating and purifying a recombinant AMV capsid protein from a transgenic plant obtained in (Step 1);
(Step 3) preparing a recombinantAMV capsid protein trimer by replacing the recombinant AMV capsid protein solution obtained in (Step 2) with a first buffer solution;
(Step 4) adding a drug to the solution of (Step 3); and
(Step 5) producing virus-like particles by replacing the first buffer solution of (Step 4) with a second buffer solution.

In an embodiment of the present invention, the first buffer solution and the second buffer solution of (Step 5) may satisfy the following features, but the present invention is not limited thereto:
(1) the first buffer solution includes 10 to 100 mM Tris and 100 to 500 mM sodium chloride, and has a pH of 6.9 to 7.5; and
(2) the second buffer solution includes 20 to 100 mM sodium pyrophosphate and 100 to 1,000 mM sodium chloride, and has a pH of 6.9 to 7.5.

In addition, the present invention provides a drug delivery system, which is prepared by the method for preparing a drug delivery system.

In addition, the present invention provides the use of a drug delivery of the recombinant AMV virus-like particles according to the present invention.

In addition, the present invention provides the use of the recombinant AMV virus-like particles for preparing a drug delivery system.

In addition, the present invention provides a method for delivering a drug, including the step of administering the drug-loaded recombinant AMV virus-like particles to a subject in need thereof.

### [Advantageous Effects]

The present invention relates to a method for efficiently producing a recombinant AMV in a plant by using a plant expression vector that is targeted to a chloroplast, and a method for producing a recombinant AMV VLP using the same. Due to the characteristics of a tertiary structure identified with high resolution, the recombinant AMV VLP accordingto the present invention can be utilized as a platform for the development of vaccines, therapeutic agents, drug delivery systems and the like. In addition, since the present invention uses a plant expression system, production costs can be dramatically reduced, and it is possible to block various contaminants (viruses, cancer genes, enterotoxins, *etc*.) at the source that may occur in conventionally known methods (methods of isolating and purifying proteins after producing the same from animal cells or microorganisms). Moreover, since the present invention includes the synthesis pathway of eukaryotic proteins that undergo post-translational modification, which is essential in animal cells, it is advantageous in that it is possible to produce proteins that maintain physiological activity, and they can be managed as seed stock, unlike animal cells or bacteria, even in the commercialization stage. Furthermore, when the demand for the corresponding material increases rapidly, it is more efficient and economically feasible compared to the existing production system using animal cells or bacteria in terms of equipment technology and cost required for mass production, and thus, it also has the advantage of being able to mass-produce and supply in a short period of time in line with demand.

### [Description of Drawings]

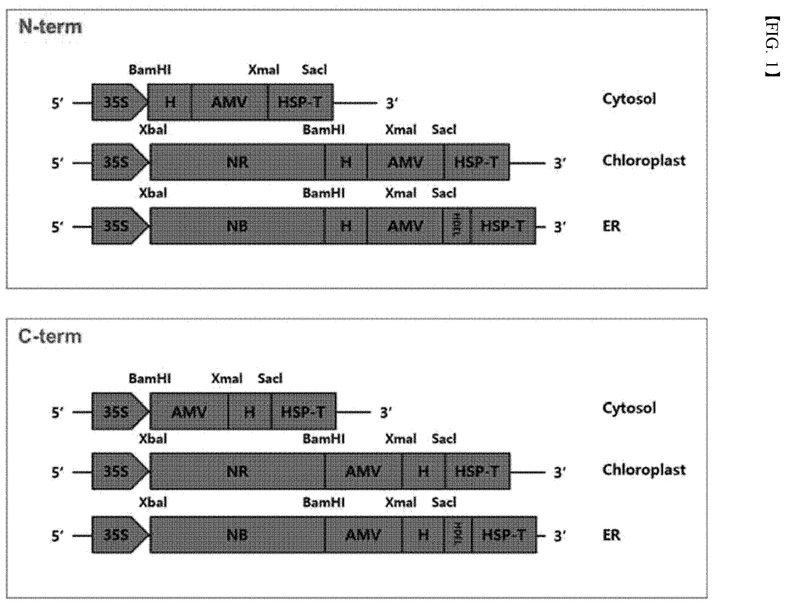

FIG. 1 is a diagram showing the expression cassettes of six recombinant AMV capsid proteins for the expression of a recombinant AMV capsid protein in a plant according to an exemplary embodiment of the present invention. The N-term (top) of FIG. 1 is a cassette of a plant expression vector designed to express the AMV capsid protein in the cytosol, endoplasmic reticulum or chloroplast in the form of attaching 6xHis (polyhistidine-tag) to theN-terminal end of a polynucleotide encoding the AMV capsid protein. The C-term (bottom) of FIG. 1 is a cassette of a plant expression vector designed to express the AMV capsid protein in the cytosol, endoplasmic reticulum or chloroplast in the form of attaching 6xHis to the C-terminal end of a polynucleotide encoding the AMV capsid protein (H, polyhistidine-tag; HSP-T, Hsp terminator; NR, Rubisco transit peptide; NB, New BiP signal peptide; HDEL, ER retention signal peptide).

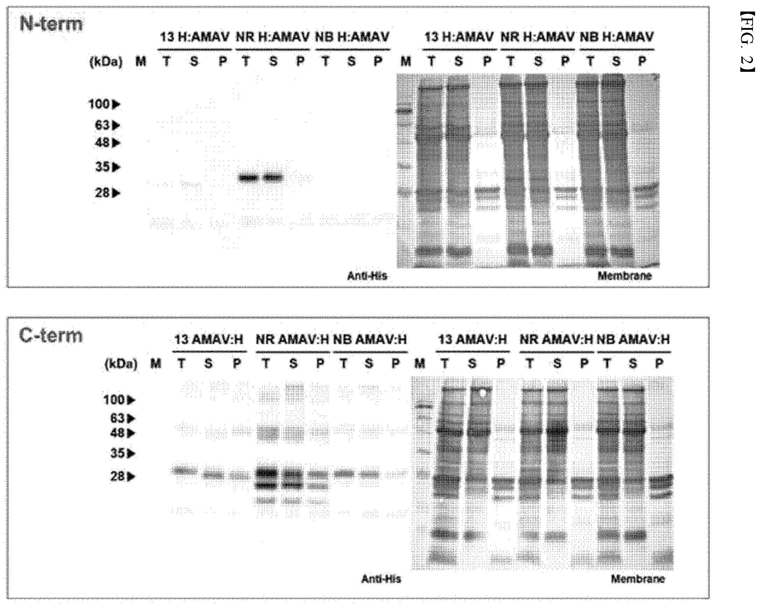

FIG. 2 is a diagram showing the results of confirming the expression of recombinant AMV capsid protein by six different plant expression vectors in a plant according to an exemplary embodiment of the present invention by western blotting.

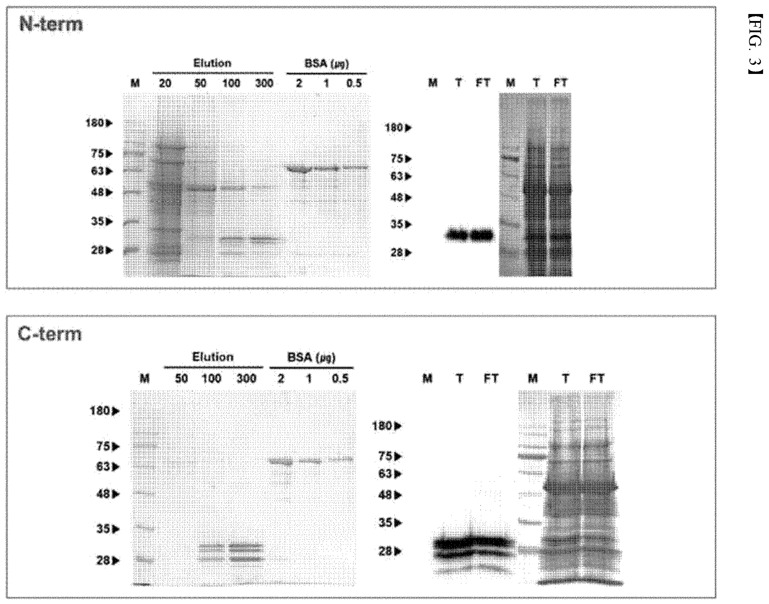

FIG. 3 is a diagram showing the results of isolation and purification by using affinity chromatography of recombinant AMV capsid protein according to an exemplary embodiment of the present invention by SDS-PAGE and Western blotting.

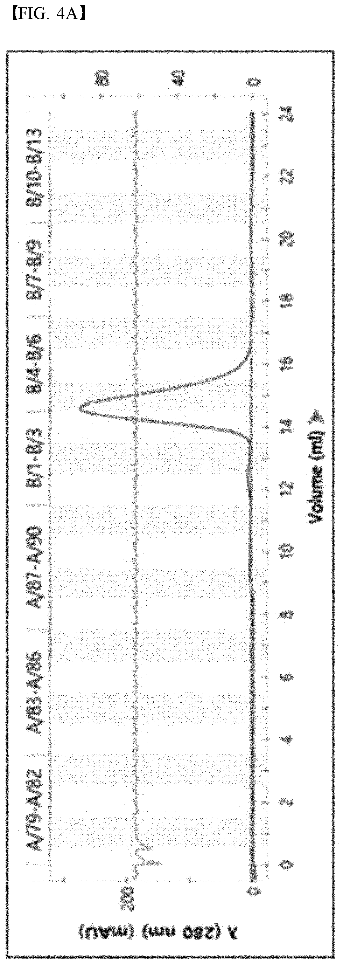

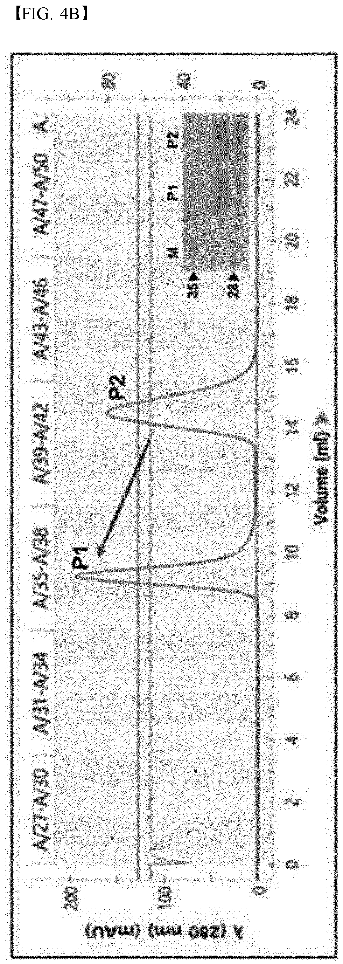

FIG. 4a to 4b are diagrams showing the results of confirming the conditions for preparing the isolated and purified recombinantAMV capsid protein in the present invention from a trimeric form to a VLP form by using size exclusion chromatography. FIG. 4a shows the result of size exclusion chromatography equilibrated with the first buffer of Example 4. FIG. 4b shows the result of size exclusion chromatography equilibrated with the second buffer of Example 4.

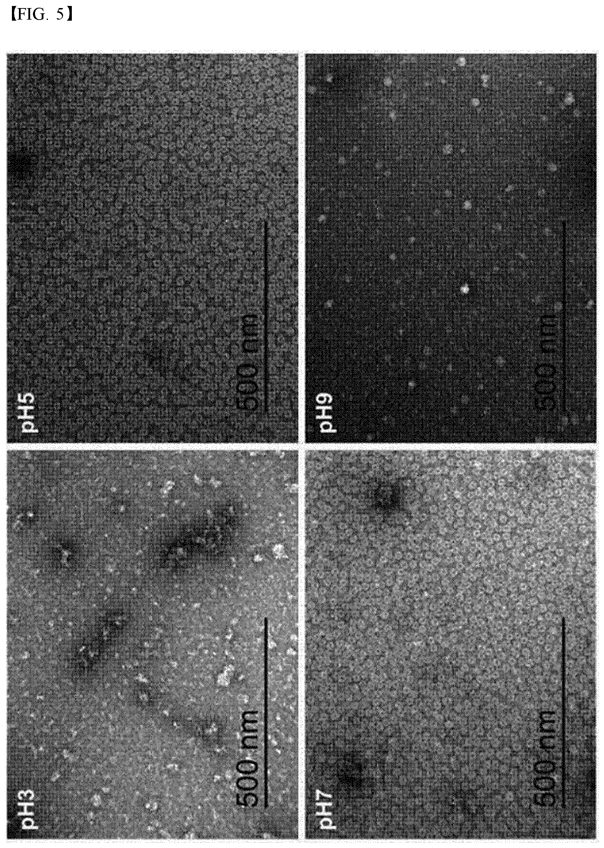

FIG. 5 is a diagram showing images taken with a transmission electron microscope by treating the isolated and purified recombinant AMV VLPs in the present invention under various pH conditions.

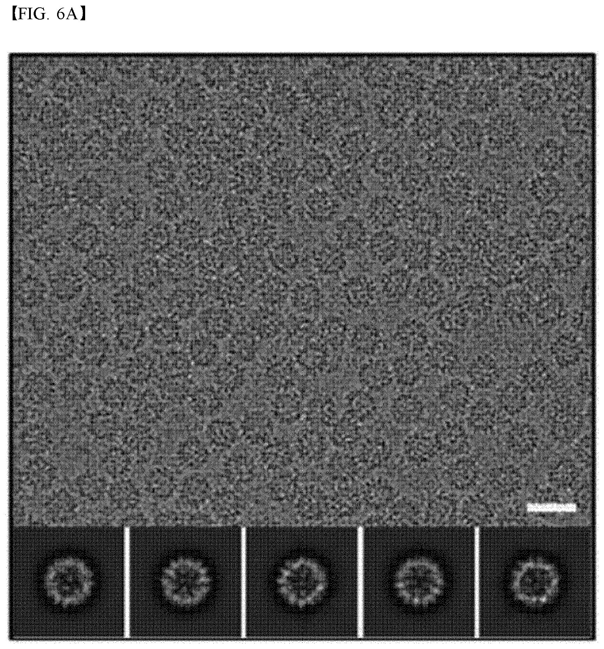

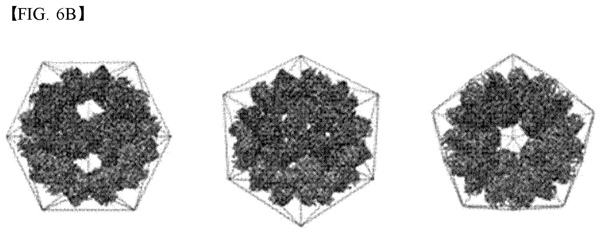

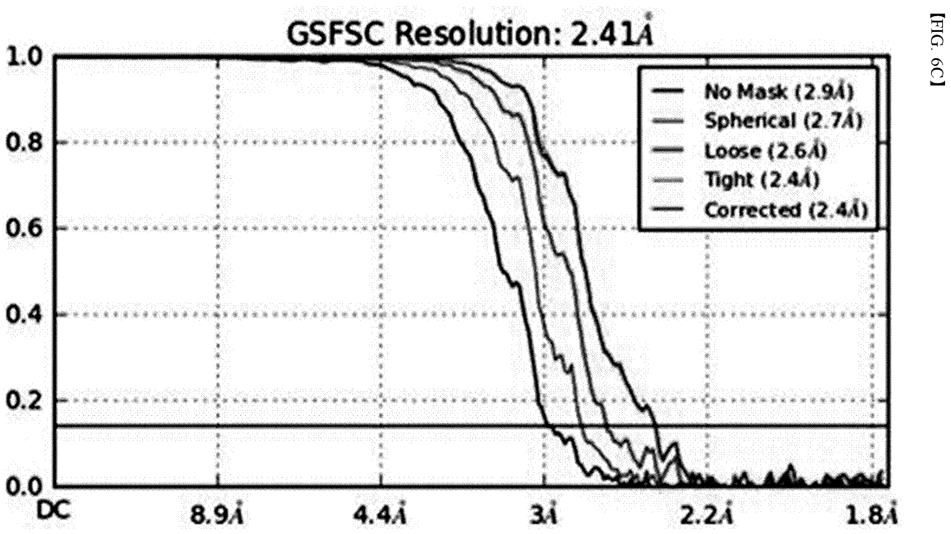

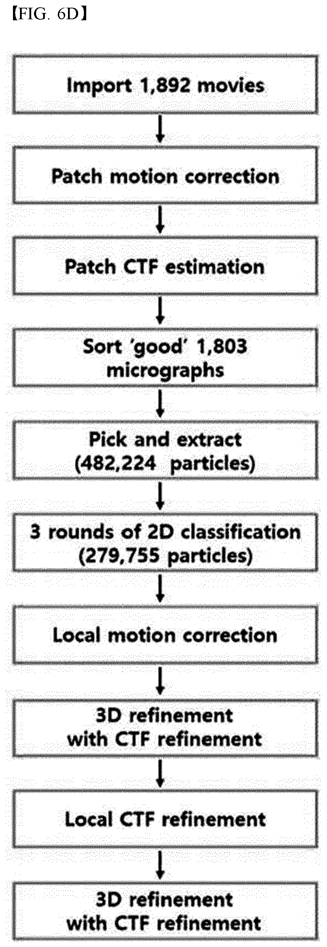

FIG. 6a to 6d are diagrams showing images taken by using Cryo-electron microscopy of the recombinant AMV VLPs isolated and purified in the present invention. FIG. 6a shows a two-dimensional (2D) class average of AMV VLPs. FIG. 6b shows an icosahedral 3D structure of AMV VLPs constructed based on the 2D class average. FIG. 6c shows the result of measuring the resolution based on the tertiary structure of the AMV VLPs. FIG. 6d is a flowchart showing the image processing of the AMV VLPs and the procedure of model building.

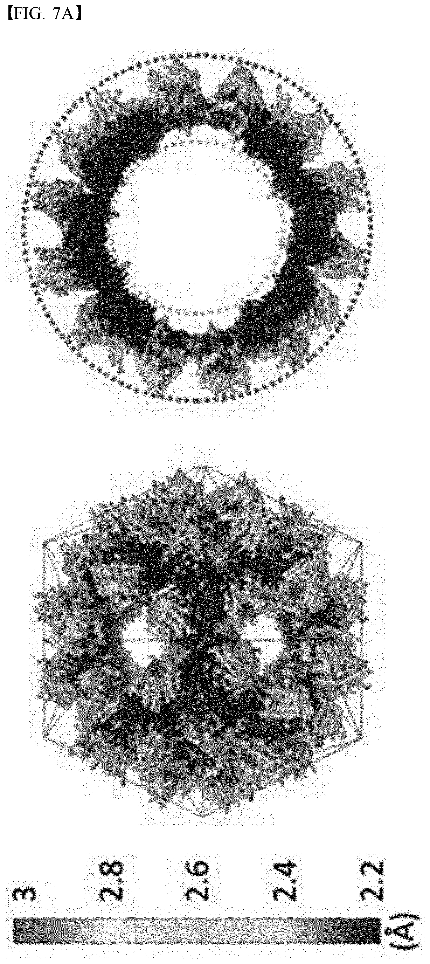

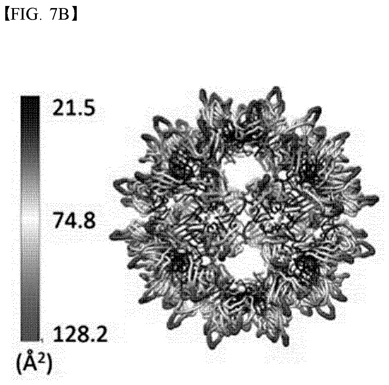

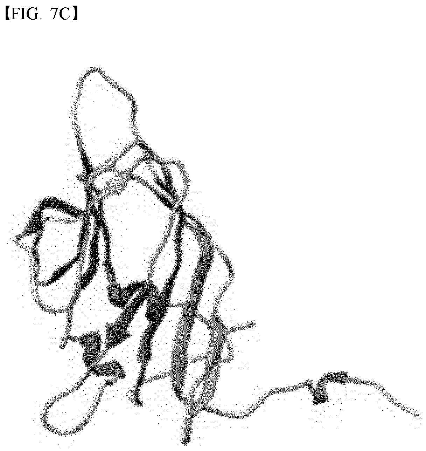

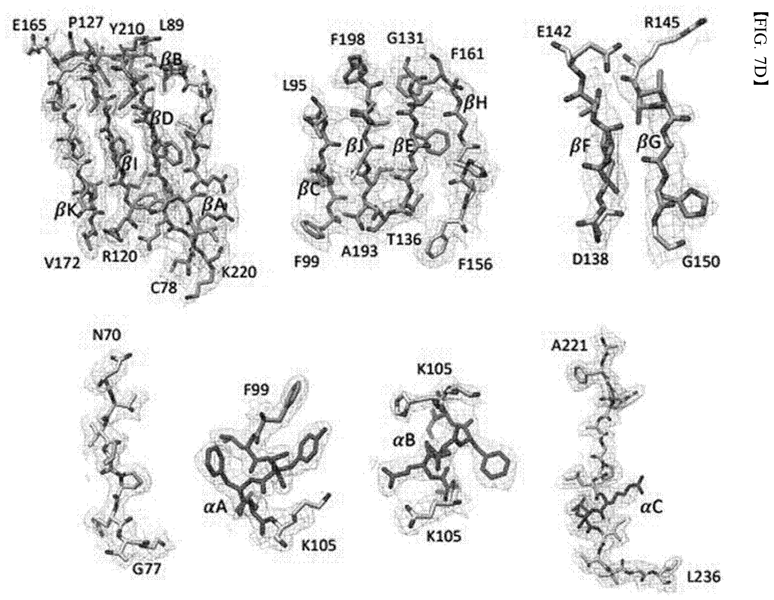

FIG. 7a to 7d are diagrams analyzing the overall structure of the recombinant AMV VLPs, the structure of the monomer and the positions of amino acids by using Cryo-electron microscopy and structural analysis software in the present invention. FIG. 7a shows the local resolution of the Cryo-EM map, and FIG. 7b shows the B-factor of the atomic model. FIG. 7c shows the structure of the recombinant AMV capsid monomer. FIG. 7d shows the sequence of amino acids in recombinantAMV capsid monomers and the electron density maps of representative amino acid residues that are analyzed at high resolution.

### [Modes of the Invention]

In the production of recombinant AMV capsid proteins that form virus-like particles, which are the target proteins, in transgenic plants, the inventors of the present invention confirmed that in order to increase the expression level of the protein in the translation stage, when a Rubisco transit peptide is fused to the target protein to be targeted to the chloroplast and a polyhistidine-tag is attached for isolation and purification, the protein expression level and isolation and purification efficiency are improved, and by using the same, it was confirmed that the target protein production efficiency can be dramatically increased in transgenic plants, and in addition, it was confirmed that the AMV capsid protein can form VLPs well by adjusting the composition of the buffer solution, thereby completing the present invention.

As such, in an exemplary embodiment of the present invention, the pCAMBIA1300 plant expression vector (1) was constructed such that the AMV capsid protein respectively fused with a polyhistidine-tag to the N-term end and the C-term ends could be expressed in the cytosol, andRbc-TP (Rubisco transit peptide) was fused to (1) such that the same form of protein could be expressed in the chloroplast, or NB (New BiP) signal peptide was fused to (1) such that it could be expressed in the endoplasmic reticulum. In addition, a total of 6 plant expression vectors were constructed by designing the AMV capsid protein to be expressed at the N-term or C-term end of the AMV capsid protein-encoding polynucleotide in each cell organelle (refer to Example 1).

In another exemplary embodiment of the present invention, after transforming the plant expression vector into Agrobacteria, it was injected into the back of the *Nicotiana benthamiana* leaf to express the recombinantAMV capsid protein in plants (refer to Example 2).

In another exemplary embodiment of the present invention, the recombinant protein was isolated and purified from the *Nicotiana benthamiana* leaf in which the recombinant AMV capsid protein is expressed by using a column filled with iminodiacetic acid (Ni-IDA) agarose resin, and the self-assembly of the AMV capsid protein was induced by adjusting the composition of the buffer solution for making virus-like particles. Specifically, it was confirmed that the AMV capsid protein formed a trimer in the first buffer solution and the AMV capsid protein assembled virus-like particles in the second buffer solution (refer to Examples 3 to 4).

In still another exemplary embodiment of the present invention, the stability of the VLP according to the pH change was confirmed by using a transmission electron microscope for the composition including the AMV virus-like particles obtained above, and it was confirmed that the VLP shape was stably maintained between pH 5 and pH 7 (refer to Example 5).

In still another exemplary embodiment of the present invention, the high-resolution tertiary structure of the AMV VLP was confirmed by using a Cryo-electron microscope (refer to Examples 6 and 7).

Accordingly, the present invention may provide a recombinant vector for expressing a plant, including a polynucleotide encoding a Rubisco transit peptide including the amino acid sequence represented by SEQ ID NO: 2, a polynucleotide encoding a polyhistidine-tag (6xHis-tag) including the amino acid sequence represented by SEQ ID NO: 6, and a polynucleotide encoding an AMV capsid protein including the amino acid sequence represented by SEQ ID NO: 4.

As used herein, the term "Rubisco transit peptide" refers to an N-terminal transit peptide of a Rubisco (Ribulose-1,5-Bisphosphate Carboxylase/Oxygenase) small subunit, and preferably, it may be encoded by a polynucleotide including the nucleotide sequence of SEQ ID NO: 1, and it may be most preferably encoded by the polynucleotide represented by SEQ ID NO: 1, but it may also be encoded by a nucleotide sequence having at least 80% sequence homology to the nucleotide sequence of SEQ ID NO: 1, and more preferably, it may be encoded by a nucleotide sequence having at least 90% sequence homology to the nucleotide sequence of SEQ ID NO: 1, and even more preferably, it may be encoded by a nucleotide sequence having at least 95% sequence homology to the nucleotide sequence of SEQ ID NO: 1. For example, it includes a polynucleotide having sequence homology of 70%, 71%, 72%, 73%, 74%, 75%, 76%, 77%, 78%, 79%, 80%, 81%, 82%, 83%, 84%, 85 %, 86%, 87%, 88%, 89%, 90%, 91%, 92%, 93%, 94%, 95%, 96%, 97%, 98%, 99% and 100%. The term "% of sequence homology" for a polynucleotide is determined by comparing two optimally aligned sequences with a comparison region, wherein a portion of the polynucleotide sequence in the comparison region may include additions or deletions (i.e., gaps) compared to a reference sequence (not including additions or deletions) for the optimal alignment of the two sequences. The Rubisco transit peptide is used to move the recombinant protein expressed in the transgenic plant into the chloroplast, and when it is expressed, a portion of the sequence may be cut off, leaving only some amino acids. More specifically, when the recombinant AMV capsid protein fused with the Rubisco transit peptide according to the present invention is expressed in a plant, the front 54 amino acids of the Rubisco transit peptide are truncated and lost, and only the amino acid sequence represented by SEQIDNO: 2 may remain. Further, in this case, an additional amino acid may be inserted between the Rubisco transit peptide sequence and the AMV capsid protein sequence in order to match the DNA frames, and preferably, glycine or isoleucine may be additionally inserted.

As used herein, the term "New BiP (NB)" is used to transfer the expressed recombinant protein to the endoplasmic reticulum, and preferably, it is a gene including the nucleotide sequence of SEQ ID NO: 9, and most preferably, it may be a gene represented by SEQ ID NO: 9, or it may include a nucleotide sequence having at least 80% sequence homology, more preferably, at least 90% sequence homology, and even more preferably, at least 95% sequence homology to the nucleotide sequence of SEQ ID NO: 9. The NB gene is truncated in its entirety when expressed, leaving no amino acids for BiP.

As used herein, the term "transit peptide" or "signal peptide" refers to an amino acid sequence which is capable of inducing the transport or localization of a protein into a specific organelle, cellular compartment or extracellular portion. This term includes both of the transit peptide and the nucleotide sequence encoding the transit peptide.

As used herein, the term "AMV" refers to an Alfalfa mosaic virus, which is an icosahedral non-enveloped DNAvirus. AMV is a virus that causes mosaic-shaped lesions in plants, but it is known to not cause any infections or lesions in humans or animals. The "AMV" in the present invention is encoded by a polynucleotide including the nucleotide sequence of SEQ ID NO: 3 or 10, and more preferably, it is encoded by the polynucleotide represented by SEQ ID NO: 3 or 10, and most preferably, it is encoded by the polypeptide represented by SEQ ID NO: 3, but it may also be encoded by a nucleotide sequence having at least 80% sequence homology, more preferably, at least 90% sequence homology, and even more preferably, at least 95% sequence homology to the nucleotide sequence of SEQ ID NO: 3 or 10.

As used herein, the term "polynucleotide" refers to an oligomer or polymer including two or more linked nucleotides or nucleotide derivatives that are linked to each other generally by phosphodiester bonds, including deoxyribonucleic acid (DNA) and ribonucleic acid (RNA). Polynucleotides also include DNA and RNA derivatives including, for example, nucleotide analogs or"backbone" bonds other than phosphodiester bonds, such as phosphotriester bonds, phosphoramidate bonds, phosphorothioate bonds, thioester bonds or peptide bonds (peptides nucleic acids). Polynucleotides include single-stranded and/or double-stranded polynucleotides, such as deoxyribonucleic acid (DNA) and ribonucleic acid (RNA), as well as analogues of either RNA or DNA.

As used herein, the term "vector" refers to a DNA preparation containing a DNA sequence which is operably linked to suitable regulatory sequences that are capable of expressing DNA in a suitable host. A vector may be a plasmid, a phage particle or simply a potential genome insert. Upon transformation into an appropriate host, the vector may replicate and function independently of the host's genome, or in some cases, it may be integrated into the genome itself. As a plasmid is currently the most commonly used form of vector, the terms "plasmid" and "vector" are sometimes used interchangeably. However, the present invention includes other forms of vectors that have an equivalent function as known or coming to be known in the art.

The Rubisco transit peptide of the present invention may include the amino acid sequence represented by SEQ ID NO: 2, and in addition, theAMV capsid protein may include the amino acid sequence represented by SEQ ID NO: 4. Further, in the amino acid sequence of the Rubisco transit peptide of the present invention, a variant of SEQ ID NO: 2 is included in the scope of the present invention. Specifically, the peptide may include an amino acid sequence having at least 90% sequence homology, more preferably, at least 95% sequence homology, and most preferably, at least 98% sequence homology to the amino acid sequence of SEQ ID NO: 2. In addition, the amino acid sequence of the AMV capsid protein of the present invention includes a variant of SEQ ID NO: 4 within the scope of the present invention. Specifically, it may include an amino acid sequence having at least 90% sequence homology, more preferably, at least 95% sequence homology, and most preferably, at least 98% sequence homology to the amino acid sequence of SEQ ID NO: 4 of the protein.

In addition, the recombinant vector of the present invention may further include a polynucleotide encoding a polyhistidine-tag including the amino acid sequence represented by SEQ ID NO: 6. The polyhistidine-tag is encoded by a polynucleotide including the nucleotide sequence represented by SEQ ID NO: 5, and most preferably, it is encoded by the polynucleotide represented by SEQ ID NO: 5.

The polyhistidine-tag is included for easy isolation in addition to AMV, which is the target protein of the present invention, and as a gene for a tag that can be substituted for the same, Avi tag, Calmodulin tag, polyglutamate tag, E tag, FLAG tag, HA tag, Myc tag, S tag, SBP tag, IgG Fc tag, CTB tag, Softag 1 tag, Softag 3 tag, Strep tag, TC tag, V5 tag, VSV tag, Xpress tag and the like may be included.

In addition, the polyhistidine-tag may be linked to the N-term end or C-term end of the polynucleotide encoding the AMV capsid protein, and preferably, it may be linked to the C-term end.

Further, in the recombinant vector of the present invention, between a promoter and a terminator, (1) a polynucleotide encoding a Rubisco transit peptide, a polynucleotide encoding an AMV capsid protein and a polynucleotide encoding a polyhistidine-tag may be sequentially linked; or (2) a polynucleotide encoding a Rubisco transit peptide, a polynucleotide encoding a polyhistidine-tag and a polynucleotide encoding an AMV capsid protein may be sequentially linked, but the present invention is not limited to the linkage order. In the case of (1), the entire insert sequence inserted into the recombinant vector is encoded by a polynucleotide including the nucleotide sequence of SEQ ID NO: 11, and most preferably, it may be encoded by the polynucleotide represented by SEQ ID NO: 11, but it may be also encoded by a nucleotide sequence having at least 80% sequence homology, more preferably, at least 90% sequence homology, and even more preferably, at least 95% sequence homology to the nucleotide sequence of SEQ ID NO: 11.

The promoter may include pEMU promoter, MAS promoter, histone promoter, Clp promoter, cauliflower mosaic virus-derived 35S promoter, cauliflower mosaic virus-derived 19S RNA promoter, plant actin protein promoter, ubiquitin protein promoter, a cytomegalovirus (CMV) promoter, a simian virus 40 (SV40) promoter, a respiratory syncytial virus (RSV) promoter, an elongation factor-1 alpha (EF-1α) promoter and the like, and most preferably, it may be a CaMV35 S promoter. The terminator may include, for example, nopaline synthase (NOS), rice amylase RAmy1 A terminator, faceolin terminator, an Octopine gene terminator of *Agrobacterium tumefaciens,* an rrnB1/B2 terminator of *Escherichia coli* and the like, and most preferably, it may be an HSP terminator. However, the examples of such promoters or terminators are merely illustrative, and the present invention is not limited thereto.

In another aspect of the presentinvention, it is possible to provide a transgenic plant, which is transformed with the recombinant vector according to the present invention.

As used herein, the term "transformation" collectively refers to a change in the genetic properties of an organism due to the injected DNA, and the term "transgenic plant" refers to a plant that is produced by injecting an external gene by the molecular genetic method, and preferably, it is a plant which is transformed by the recombinant expression vector of the present invention, and there is no limitation for the plant as long as it is intended to achieve the objects of the present invention.

In addition, the transgenic plant according to the present invention may be produced by methods such as transformation, transfection, Agrobacterium-mediated transformation method, particle gun bombardment, sonication, electroporation or PEG (Polyethylene glycol)-mediated transformation, but there is no limitation as long as it is a method that is capable of injecting the vector of the present invention.

In another aspect of the present invention, the present invention provides a method for isolating and purifying a recombinantAMV capsid protein, including the following steps:
(Step 1)transforming a plant by using the recombinant vector according to the present invention;
(Step 2) preparing a plant mixture solution by mixing the transgenic plant obtained in (Step 1) with a protein extraction buffer solution;
(Step 3) recovering a mixed solution from which plant debris is removed from the mixture solution obtained in (Step 2);
(Step 4) injecting the mixture solution obtained in (Step 3) into an agarose-filled column to adsorb a polyhistidine-tagged recombinant AMV capsid protein to the agarose;
(Step 5) washing by injecting a washing solution into the column; and
(Step 6) eluting the recombinant protein adsorbed on agarose by injecting an elution solution into the column.

In addition, the present invention provides a recombinant AMV capsid protein that is eluted by the above isolation and purification method.

The protein extraction buffer solution according to the present invention may include 10 to 100 mMTris, 100 to 1,500 mMmagnesium chloride (MgCl₂), 0.01 to 1% Triton X-100 (polyethylene glycol p-(1,1,3, 3-tetramethylbutyl)-phenylether) and 5 to 300 mM imidazole. More preferably, the protein extraction buffer solution may include 10 to 50 mM Tris, 500 to 1200 mMMgCl₂, 0.01 to 0.7% Triton X-100 and 5 to 100 mM imidazole. Most preferably, the protein extraction buffer solution may include 20 mM Tris, 1 M MgCl₂, 0.5% Triton X-100 and 10 mM imidazole. In the present invention, Tris may be a substance that is titrated to pH 8.0 with HCl (Tris-HCl).

In addition, the protein extraction buffer solution according to the present invention may have a pH of 7 to 9, and preferably, a pH of 7.5 to 8.5. Most preferably, the protein extraction buffer solution may have a pH of 8.

The agarose according to the present invention may be iminodiacetic acid (Ni-IDA) agarose.

Further, in the present invention, (Step 1) may be to transform a plantby using bacteria into which the recombinant vector is introduced, and the bacteria may be preferably *Agrobacterium tumefaciens.*

In the present invention, the plant may be a dicotyledonous plant selected from the group consisting of Arabidopsis, soybean, tobacco, eggplant, pepper, potato, tomato, Chinese cabbage, cabbage and lettuce; or a monocotyledonous plant selected from the group consisting of rice, barley, wheat, rye, corn, sugar cane, oat and onion,

As used herein, the term "plant" may be used without limitation as long as it is a plant that is capable of mass-producing the recombinant protein of the present invention, but more specifically, it may be selected from the group of plants listed above, and preferably, it may be tobacco. The tobacco in the present invention is a plant of the *Nicotiana* genus, and it is not particularly limited in type as long as it can overexpress a protein, and the present invention may be practiced by selecting an appropriate variety according to the transformation method and the purpose of mass production of protein. For example, varieties such as *Nicotiana benthamiana L.* or *Nicotiana tabacum cv. Xanthi* may be used.

As used herein, the term "capsid" refers to a protein constituting the coat of a virus. Naturally, the capsid self-assembles to form a virus shell, and the genome of the virus is packaged within the shell. Virus-like particles consist only of the capsid without the viral genome. The recombinant AMV capsid protein according to the present invention may exist individually (monomers), exist in the multimeric (e.g. trimeric) state, or it may exist in the self-assembled state into virus-like particles. In addition, the trimer of the recombinant AMV capsid protein according to the present invention may have a molecular weight of about 100 kDa in size-exclusion chromatography. Throughout the present specification, the term "about" means ±10%. Therefore, a molecular weight of about 100 kDa means 90 kDa to 110 kDa.

In addition, the present invention provides a method for producing recombinant AMV virus-like particles (VLP), including the following steps:
(Step 1)transforming a plantby using the recombinant vector according to the present invention;
(Step 2) preparing a plant mixture solution by mixing a transgenic plant obtained in (Step 1) with a protein extraction buffer solution;
(Step 3) recovering a mixture solution from which plant debris is removed from the mixture solution obtained in (Step 2);
(Step 4) injecting the mixture solution obtained in (Step 3) into an agarose-filled column to adsorb a polyhistidine-tagged recombinant AMV capsid protein to the agarose;
(Step 5) washing by injecting a washing solution into the column;
(Step 6) injecting an elution solution into the column to elute the recombinant protein adsorbed on the agarose; and
(Step 7) preparing a protein eluted in (Step 6) into virus-like particles.

In addition, the present invention provides recombinant AMV virus-like particles, which are produced by the above production method.

The recombinant AMV virus-like particles may have a molecular weight of about 2,000 kDa or more in size-exclusion chromatography.

In addition, the recombinant AMV virus-like particles may have a spherical or cyclic shape with a diameter of 10 nm or more to 40 nm or less, and preferably, 20 nm or more to 30 nm or less, when observed with a transmission electron microscope after staining with a negative staining method.

In addition, when the recombinant AMV virus-like particles are observed with a cryogenic transmission electron microscope and the 3-dimensional structure is reconstructed, they may be spherical VLPs (3×20) in which a total of 20 trimeric complexes are assembled through a regular arrangement.

In the present invention, (Step 7) may be to produce virus-like particles by replacing the protein extraction buffer solution including the recombinantAMV capsid protein with a buffer solution for virus-like particle assembly. That is, the recombinant AMV capsid protein is self-assembled into virus-like particles in the buffer solution for assembling virus-like particles.

Specifically, the method for producing virus-like particles may include the step of replacing the protein extraction buffer solution with a buffer solution for virus-like particle assembly by using a filter and the step of concentrating such that the recombinant AMV capsid protein can form virus-like particles by self-assembly.

In the present invention, the buffer solution for virus-like particle assembly may include 20 to 100 mM sodium pyrophosphate and 100 to 1,000 mM sodium chloride (NaCl). Preferably, the buffer solution may include 30 to 70 mM sodium pyrophosphate and 300 to 700 mM NaCl. Most preferably, the buffer solution may include 50 mM sodium pyrophosphate and 500 mM NaCl. In the present specification, the term "buffer solution for virus-like particle assembly" is used interchangeably with "a second buffer solution."

In addition, the pH of the buffer solution may be pH 6.9 to 7.5, preferably, pH 6.9 to 7.2, and most preferably, pH 7.

In addition, the method for producing virus-like particles may further include the step of performing size exclusion chromatography to purify the self-assembled recombinant AMV virus-like particles.

In addition, the present invention provides a method for preparing a vaccine composition including the recombinant AMV virus-like particles, including the following steps:
(Step 1)transforming a plant by using the recombinant vector according to the present invention;
(Step 2) isolating and purifying a recombinant AMV capsid protein from a transgenic plant obtained in (Step 1);
(Step 3) producing virus-like particles from the recombinant AMV capsid protein obtained in (Step 2);
(Step 4) identifying the 3D structure of the virus-like particles obtained in (Step 3) by using a Cryo-electron microscope; and
(Step 5) preparing a vaccine composition including the virus-like particles.

That is, the present invention provides a vaccine composition including the recombinant AMV virus-like particles according to the present invention and an antigen protein as active ingredients.

In the present invention, "vaccine" is a biological agent containing an antigen that induces an immune response in a living body, and it refers to an immunogen that induces immunity in a living body by injecting or orally administering to a person or animal for the prevention of infection. The animal is a human or non-human animal, and the non-human animal refers to a pig, a cow, a horse, a dog, a goat, a sheep and the like, but the present invention is not limited thereto.

The "vaccine composition" or "drug delivery system" of the present invention may be used by formulating in the form of oral dosage forms and sterile injection solutions such as powders, granules, tablets, capsules, suspensions, emulsions, syrups, aerosols and the like, respectively, according to conventional methods. In the case of preparation, it may be prepared by using commonly used diluents or excipients such as fillers, extenders, binders, wetting agents, disintegrants, surfactants and the like. Solid preparations for oral administration include tablets, pills, powders, granules, capsules and the like, and these solid preparations may be prepared by mixing the lecithin-like emulsifier with at least one excipient, for example, starch, calcium carbonate, sucrose or lactose, gelatin and the like. In addition to simple excipients, lubricants such as magnesium stearate talc may also be used. As liquid preparations for oral administration, suspensions, internal solutions, emulsions, syrups and the like may be used, and in addition to commonly used simple diluents such as water and liquid paraffin, various excipients such as wetting agents, sweeteners, fragrances, preservatives and the like may be included. Preparations for parenteral administration include sterile aqueous solutions, non-aqueous preparations, suspensions, emulsions and freeze-dried preparations. As the non-aqueous preparation and suspension, propylene glycol, polyethylene glycol, vegetable oils such as olive oil, and injectable esters such as ethyl oleate may be used.

The route of administration of the vaccine composition or drug delivery system according to the present invention is preferably, but not limited to, oral, intravenous, intramuscular, intraarterial, intramedullary, intrathecal, intracardiac, transdermal, subcutaneous, intraperitoneal, intranasal, intestinal, topical, sublingual or rectal administration. Oral or parenteral administrationis preferred. As used herein, the term "parenteral" includes subcutaneous, intradermal, intravenous, intramuscular, intraarticular, intrabursar, intrasternal, intrathecal, intralesional and intracranial injection or infusion techniques. The vaccine composition of the present invention may also be administered in the form of a suppository for rectal administration.

The dosage of the vaccine composition, pharmaceutical composition or drug delivery system according to the present invention is selected in consideration of the subject's age, weight, gender, physical condition and the like. The amount required to induce an immunoprotective response in a subject without significant side effects may vary depending on the recombinant protein used as the immunogen and any presence of excipients.

In the present invention, the method for preparing a vaccine composition may further include the step of adding an adjuvant.

In the present invention, "adjuvant" refers to a substance or composition that is added to a vaccine or pharmaceutically active ingredients to increase or affect an immune response. Typically, it usually means a carrier or auxiliary substance for an immunogen and/or other pharmaceutically active substance or composition. Typically, the term "adjuvant" is to be interpreted in a broad sense and means a broad range of sub stances or strategies that are capable of enhancing the immunogenicity of an antigen incorporated into or administered with the adjuvant. In addition, the adjuvant, without being limited thereto, may be divided into an immune potentiator, an antigen delivery system or a combination thereof.

In addition, the method for preparing a vaccine composition may satisfy one of the following characteristics: (1) the recombinant vector of (Step 1) further includes a polynucleotide encoding an antigen protein, and the polynucleotide encoding the antigen protein is linked to the N-term end of a polynucleotide encoding an AMV capsid protein; or (2) (Step 5) includes the step of conjugating an antigen protein to a surface of the virus-like particles.

In the present invention, "antigen protein" refers to a protein for inducing the production of antibodies when the vaccine composition according to the present invention is injected into the body. The antigen protein may be a pathogenic protein or a recombinant protein, but any protein that is capable of inducing antibody production may be included without limitation. The antigen protein is displayed on the surface of the vaccine composition according to the present invention. When the polynucleotide encoding the antigen protein is linked to the N-term end of the polynucleotide encoding the AMV capsid protein, the antigen protein is expressed together with the recombinant AMV capsid protein from the recombinant vector according to the present invention and assembled together such that it is displayed on the surface upon the virus-like particle assembly of the capsid protein. Alternatively, the antigen protein may be displayed in the vaccine composition by binding the antigen protein to the surface after the recombinant AMV virus-like particles are produced according to the present invention. The method for binding a foreign protein to the surface of virus-like particles may be applied without limitation as long as it is known in the art.

In addition, the present invention provides a vaccine composition which is prepared by the method for preparing a vaccine composition.

The vaccine composition may further include an adjuvant. In addition, the adjuvant may be alum, but any kind of aluminum salt suitable for use as an adjuvant may be used in the present invention. Example of aluminum salt include aluminum hydroxide (Al(OH)₃), aluminum phosphate (AlPO₄), aluminum hydrochloride, aluminum sulfate, ammonium alum, potassium alum or aluminum silicate. Preferably, aluminum hydroxide or aluminum phosphate may be used as an aluminum salt adjuvant.

In addition, the present invention provides a method for preparing a drug delivery system including recombinant AMV virus-like particles, including the following steps:
(Step 1)transforming a plantby using the recombinant vector according to the present invention;
(Step 2) isolating and purifying a recombinant AMV capsid protein from a transgenic plant obtained in (Step 1);
(Step 3) preparing a recombinantAMV capsid protein trimer by replacing the recombinant AMV capsid protein solution obtained in (Step 2) with a first buffer solution;
(Step 4) adding a drug to the solution of (Step 3); and
(Step 5) producing virus-like particles by replacing the first buffer solution of (Step 4) with a second buffer solution.

The drug delivery system according to the present invention includes a drug in the recombinant AMV virus-like particles according to the present invention to deliver the drug to a target point via the virus-like particles.

The drug may include all types of drugs such as anticancer drugs without limitation, and two or more drugs may be simultaneously supported on the virus-like particles according to the present invention.

In the method for preparing a drug delivery system according to the present invention, the first buffer and the second buffer of (Step 5) may satisfy the following features: (1) the first buffer solution includes 10 to 100 mM Tris and 100 to 500 mM sodium chloride, and has a pH of 6.9 to 7.5; and (2) the second buffer solution includes 20 to 100 mM sodium pyrophosphate and 100 to 1,000 mM sodium chloride, and has a pH of 6.9 to 7.5.

Specifically, when the recombinant vector according to the present invention is transformed into a plant to isolate and purify the recombinantAMV capsid protein with a protein extraction buffer solution and then replace the buffer solution with the first buffer solution, the recombinant AMV capsid protein monomers form a trimer. The first buffer solution may include 10 to 100 mMTris and 100 to 500 mM sodium chloride (NaCl). Preferably, the buffer solution may include 10 to 50 mM Tris and 200 to 400 mMNaCl. Most preferably, the buffer solution may include 25 mMTris, and 300 mM NaCl. In the present invention, Tris may be titrated with HCl (Tris-HCl).

Subsequently, after adding a drug to the recombinant AMV capsid protein trimer, when the first buffer solution is replaced with the second buffer solution, and the drug-loaded recombinant AMV virus-like particles are prepared. The drug may be included inside the virus-like particles or bound to the surface of the virus-like particle, but the present invention is not limited to the location. The second buffer solution may include 20 to 100 mM sodium pyrophosphate and 100 to 1,000 mM sodium chloride (NaCl). Preferably, the buffer solution may include 30 to 70 mM sodium pyrophosphate and 300 to 700 mM NaCl. Most preferably, the buffer solution may include 50 mM sodium pyrophosphate and 500 mM NaCl.

In addition, the pH of the first buffer solution and the second buffer solution may be pH 6.9 to 7.5, preferably, pH 6.9 to 7.2, and most preferably, pH 7.

The terms or words used in the present specification and claims should not be construed as being limited to their ordinary or dictionary meanings, and based on the principle that the inventor can appropriately define the concept of the term s in order to explain his or her invention in the best way, they should be interpreted as meanings and concepts that are consistent with the technical spirit of the present invention.

Hereinafter, preferred examples are presented to help the understanding of the present invention. However, the following examples are only provided for easier understanding of the present invention, and the contents of the present invention are not limited by the following examples.

### [Example]

### Example 1: Construction of plant expression vector for expressing recombinant AMV capsid protein

As shown in the cleavage map of FIG. 1, a vector for expressing a recombinant plant was constructed such that the recombinant AMV capsid protein could be expressed in the plant.

More specifically, the gene information on the AMV capsid protein was obtained, and the gene (SEQ ID NO: 4) was synthesized with a sequence optimized for expression in *Nicotiana benthamiana.* In order to confirm the expression pattern of the AMV capsid protein according to the location of the polyhistidine-tag (6xHis-tag) and the target organelle, six expression vectors were constructed as follows.

The recombinantAMV capsid protein plant expression vector **targeted to the cytosol** was constructed by sequentially linking a polynucleotide (SEQ ID NO: 5) encoding 6 consecutive histidines at the front (N-term) or the back (C-term), respectively, with a polynucleotide (SEQ ID NO: 3) encoding an AMV capsid protein in the middle, between the CaMV3 5 S promoter gene and the Hsp terminator of the pCAMBIA1300 vector.

The recombinantAMV capsid protein plant expression vector **targeted to the chloroplast** was constructed by sequentially linking a polynucleotide encoding a Rubisco transit peptide (SEQ ID NO: 1) and a polynucleotide encoding an AMV capsid protein (SEQ ID NO: 3) between the CaMV35S promoter gene and the Hsp terminator of the pCAMBIA1300 vector. In this case, in order to distinguish the N-term polyhistidine-tag from the C-term polyhistidine-tag, a polynucleotide (SEQ ID NO: 5) encoding 6 consecutive histidines was inserted before or after the AMV, respectively.

The recombinantAMV capsid protein plant expression vector targeted to **the endoplasmic reticulum** was constructed by sequentially linking a polynucleotide encoding a New BiP (NB) signal peptide (SEQ ID NO: 9), a polynucleotide encoding an AMV capsid protein (SEQ ID NO: 3) and a His-Asp-Glu-Leu (HDEL) peptide encoding Polynucleotide (SEQ ID NO: 7) between the CaMV35 S promoter gene and the Hsp terminator of the pCAMBIA1300 vector. In this case, in order to distinguish the N-term polyhistidine-tag from the C-term polyhistidine-tag, a polynucleotide (SEQ ID NO: 5) encoding six consecutive histidines was inserted before or after the AMV, respectively.

### Example 2: Confirmation of expression of recombinant AMV capsid protein

### 2.1. Transient expression of plant expression vector

The plant expression vector prepared in Example 1 was transformed into the Agrobacterium LBA4404 strain by using the electrophoresis method. After culturing the transformed agrobacteria in 5 mL of YEP liquid medium (yeast extract 10 g, peptone 10 g, NaCl 5 g, kanamycin 50 mg/L, rifampicin 25 mg/L) for 16 hours at28°C with shaking, 1 mL of the primary culture medium was inoculated into 50 mL of new YEP medium and cultured with shaking for 6 hours at 28°C. After the agrobacteria cultured in this way were collected by centrifugation (7,000 rpm, 4°C, 5 minutes), it was suspended in an infiltration buffer [10 mM MES (pH 5.7), 10 mM MgCl₂, 200 µM acetosyringone] such that the absorbance (O.D) value was 1.0 at a wavelength of 600nm. Agro-infiltration was performed by injecting the Agrobacterium suspension into the back of the *Nicotiana benthamiana* leaf by using a syringe with the needle removed.

### 2.2. Confirmation of expression of recombinant AMV capsid protein in transgenic plant

After extracting the protein from the plant leaf prepared in Example 2.1 and centrifuging the same, the protein in the water-soluble fraction (Supernatant; S), the protein in the pellet (Pellet; P) fraction and the fraction including both of the water-soluble fraction and the pellet (Total; T) were isolated from each other to confirm the expression of the recombinant AMV capsid protein by Western blotting. More specifically, 30 µL of each fraction was mixed with SDS sample buffer and then heated. Next, by electrophoresis on a 10% SDS-PAGEgel, protein bands that were separated by size were confirmed and transferred to a PVDF membrane, and after a blocking step using 5% skim milk, the expression of the recombinantAMV capsid protein was confirmed by binding an antibody reactive with polyhistidine and treating the ECL solution according to the method provided by the manufacturer.

As a result, as shown in FIG. 2, it was confirmed that the recombinantAMV capsid protein fused with the Rubisco transit peptide to be targeted to the chloroplast was expressed with high efficiency, and more than 90 to 95% of the expressed recombinant AMV capsid protein was confirmed in the aqueous fraction, and about 5 to 10% of the AMV capsid protein was observed in the pellet fraction. In comparison, the recombinant AMV capsid protein targeted to the cytosol and endoplasmic reticulum showed lower expression efficiency than the chloroplast-targeted constructs. Accordingly, the following examples were performed by using a recombinant AMV capsid protein (His-tag attached to the N-term end or C-term end) that was fused with the Rubisco transit peptide and targeted to the chloroplast.

### Example 3: Isolation and purification of recombinant AMV capsid protein expressed in transgenic plant

100 mL of a protein extraction solution [20 mMTris -HCl, pH8.0, 1M MgCl₂, 10 mM imidazole, 0.5% Triton X-100] was added to 50 g of *each Nicotiana benthamia* leaf expressing the recombinantAMV capsid protein fused with a polyhistidine-tag at the N-term end or C-term end prepared in Example 2.1, and the tissue was disrupted with a blender and centrifuged at 13,000 rpm at 4°C for 30 minutes to recover the protein extract, respectively. For isolation and purification of the recombinant AMV capsid protein from the protein extract under the above two conditions (polyhistidine-tag fused to N-term end or C-term end), affinity chromatography was simultaneously performed with a column filled with iminodiacetic acid (Ni-IDA) agarose resin. After filling the column with 10 mL of the resin, it was equilibrated with 100 mL of a washing solution [20 mM Tris-HCl, pH 8.0, 1 M MgCl₂, 10 mM Imidazole]. Each of the recovered protein extracts were applied to two different columns and equilibrated, and then, the resin was washed by flowing 100 mL of the washing solution, and the recombinant AMV capsid protein was eluted with an elution solution (20 mM Tris-HCl, pH 8.0, 1 M MgCl₂, 50 mM or 100 mM or 300 mM Imidazole). The elution solution including each recombinant AMV capsid protein was confirmed through electrophoresis (SDS-PAGE) followed by Coomassie staining and western blot experiments.

As a result, as shown in FIG. 3, the recombinant AMV capsid protein fused with a His-tag to the C-term end was confirmed to have higher purification and final recovery rates than the recombinantAMV capsid protein fused to the N-term end with a His-tag. Accordingly, the following examples were performed by using the recombinant AMV capsid protein in which a His-tag was fused to the C-term end.

### Example 4: Confirmation of VLP formation conditions of recombinant AMV capsid protein

Recombinant AMV capsid proteins form recombinant AMV virus-like particles through self-assembly. Therefore, size exclusion chromatography was performed to test and confirm the conditions for the formation of recombinantAMV virus-like particles.

5 mg of an elution solution including the isolated and purified recombinant AMV capsid protein recovered in Example 3 was dialyzed with a first buffer solution (25 mM Tris-HCl, pH 7.0, 300 mMNaCl), and then, one peak fraction was obtained by loading onto a size exclusion chromatography column equilibrated with the first buffer solution. As a result of calculating the molecular weight by using a standard size marker, a trimer structure of about 100 kDa was confirmed (FIG. 4a).

In addition, 5 mg of an elution solution including the recombinant AMV capsid protein recovered in Example 3 was dialyzed with a second buffer solution (50 mM Sodium pyrophosphate, pH 7.0, 500 mM NaCl), and then, by loading onto a size exclusion chromatography column equilibrated with the second buffer solution, it was confirmed that there was a change in the size of the AMV capsid protein (FIG. 4b). Compared to the first buffer solution, whereas the existing peak (P2) was reduced under the conditions of the second buffer solution, it was confirmed that a new peak (P1) was formed, and it was confirmed that both of P1 and P2 were the recombinant AMV capsid proteins by SDS-PAGE.

### Example 5: Identification of recombinant AMV virus-like particles

Negative-staining was performed by diluting the recombinant AMV capsid protein included in the P1 fraction that was isolated by size exclusion chromatography in the second buffer solution (50 mM Sodium pyrophosphate, pH 7.0, 500 mMNaCl) to a final concentration of 100 nM. After 5 µL of a protein solution was reacted on a carbon-coated copper grid for 1 minute, the protein solution was removed by using filter paper and washed 3 times with tertiary distilled water. In this case, the carbon-coated copper grid reacted with plasma for 1 minute to convert hydrophobic properties to hydrophilic properties such that proteins were well attached to carbon. Finally, after reacting with 1% uranyl acetate for 1 minute, negative-staining was performed, and then, the staining solution was removed with filter paper and dried at room temperature for 12 hours. The prepared grid was imaged at a magnification of20,000 times by using a transmission electron microscope (Tecnai T10, Philips) to obtain images. As a result, as shown in FIG. 5 (pH 7.0), virus-like particles composed of the recombinant AMV capsid protein could be observed.

Further, in a test where the pH of the buffer solution was changed to various pH (3.0, 5, 0, 9.0) conditions to confirm the stability of the VLP according to changes in the pH condition, the stability of the VLP was decreased under the conditions of pH 3.0 and pH 9.0, whereas it was confirmed that the stability of the VLP was maintained under the conditions of pH 5.0 and pH 7.0, and the results are shown in FIG. 5.

### Example 6: Structure of recombinant AMV capsid protein

### 6.1. Sample vitrification and data collection

In order to analyze the VLP structure of the recombinantAMV capsid protein identified in Example 5, Cryo-electron microscopy was used. After applying 3 µL of the recombinantAMV capsid protein VLPto negatively glow-discharged holey carbon grid (Quantifoil R1.2/1.3 Cu 200 mesh, SPI) at 15 mA for 1 minute, it was subjected to blotting for 6.5 seconds in the VitrobotMark IV device under the conditions of 4°C and 100% humidity, itwas quickly fixed by immersion in liquid ethane thatwas cooled with liquid nitrogen. Cryo-EM images of AMV VLPs were confirmed by using the Falcon 3EC direct electron detector (Thermo Fisher Scientific, USA) on a 300 kV Titan Krios (Thermo Fisher Scientific, USA) transmission electron microscopy, and were collected by automatic data acquisition software (EPU; Thermo Fisher Scientific, USA) (refer to FIG. 6a).

### 6.2. Image processing and model building for analysis of AMV VLP tertiary structure

The cryoSPARC v2 software was used to analyze the VLP images of the recombinant AMV capsid protein collected in Example 6.1 above. Beam-induced motion correction and dose weighting were corrected by using motion correction included in the software, and then, CTF variables were measured by using patch CTF (contrast transfer function). Since images with motion drift or images with too high or low defocus could not be used for image processing, they were visually checked and removed, and 482,224 AMV VLPs were extracted from 1,803 images to produce a 2D class average image. By using this image as a template, images with low correlation were further removed, 279,755 AMV VLPs were finally selected, and a new 2D class average was produced by using the same (bottom of FIG. 6a). The icosahedral 3D structure was reconstructedbased on the novel 2D image (refer to FIG. 6b), and the results of measuring the resolution based on the AMV VLP tertiary structure are shown in FIG. 6c, and the high-resolution structure of 2.41 Å was obtained. The process of image processing and model building is shown as a flow chart in FIG. 6d. In addition, the results of analyzing the overall structure of the recombinantAMV VLP, the structure of the monomer and the positions of amino acids using Cryo-electron microscopy and structural analysis software are shown in FIGS. 7a to 7d.

The foregoing description ofthe present invention is for illustration, and those of ordinary skill in the art to which the present invention pertains will understand that it can be easily modified into other specific forms without changing the technical spirit or essential features of the present invention. Therefore, it should be understood that the exemplary embodiments described above are illustrative in all respects and not restrictive.

### [Industrial Applicability]

The present invention relates to a method for efficiently producing a recombinant AMV in a plant by using a plant expression vector that is targeted to a chloroplast, and a method for producing a recombinant AMV VLP using the same. Due to the characteristics of a tertiary structure identified with high resolution, the recombinantAMV VLP accordingto the present invention canbe utilized as a platform for the development of vaccines, therapeutic agents, drug delivery systems and the like. In addition, since the present invention uses a plant expression system, production costs can be dramatically reduced, and it is possible to block various contaminants (viruses, cancer genes, enterotoxins, *etc*.) at the source that may occur in conventionally known methods (methods of isolating and purifying proteins after producing the same from animal cells or microorganisms). Moreover, since the present invention includes the synthesis pathway of eukaryotic proteins that undergo post-translational modification, which is essential in animal cells, it is advantageous in that it is possible to produce proteins that maintain physiological activity, and they can be managed as seed stock, unlike animal cells or bacteria, even in the commercialization stage. Furthermore, when the demand for the corresponding material increases rapidly, it is more efficient and economically feasible compared to the existing production system using animal cells or bacteria in terms of equipment technology and cost required for mass production, and thus, it also has the advantage of being able to mass-produce and supply in a short period of time in line with demand. Therefore, the present invention is expected to be advantageously utilized in various industrial fields utilizing virus-like particles, including vaccine and drug delivery technologies.

## Claims

1. A recombinant vector for expressing a plant, comprising:
a polynucleotide encoding a Rubisco transit peptide comprising the amino acid sequence represented by SEQ ID NO: 2; and
a polynucleotide encoding an Alfalfa mosaic virus (AMV) capsid protein comprising the amino acid sequence represented by SEQ ID NO: 4.

2. The recombinant vector of claim 1, wherein the polynucleotide encoding a Rubisco transit peptide comprises the nucleotide sequence represented by SEQ ID NO: 1, and the polynucleotide encoding an AMV capsid protein comprises the nucleotide sequence represented by SEQ ID NO: 3 or 10.

3. The recombinant vector of claim 1, wherein the recombinant vector further comprises a polynucleotide encoding a polyhistidine-tag comprising the amino acid sequence represented by SEQ ID NO: 6.

4. The recombinant vector of claim 3, wherein the polyhistidine-tag is linked to the C-term end or the N-term end of the polynucleotide encoding an AMV capsid protein.

5. The recombinant vector of claim 3, wherein in the recombinant vector, between a promoter and a terminator,
(1) a polynucleotide encoding a Rubisco transit peptide, a polynucleotide encoding an AMV capsid protein and a polynucleotide encoding a polyhistidine-tag are sequentially linked; or
(2) a polynucleotide encoding a Rubisco transit peptide, a polynucleotide encoding a polyhistidine-tag and a polynucleotide encoding an AMV capsid protein are sequentially linked.

6. A transgenic plant, which is transformed with the recombinant vector according to any one of claim 1 to 5.

7. A method for isolating and purifying a recombinant AMV capsid protein, comprising the following steps:
(Step 1) transforming a plant by using the recombinant vector according to any one of claims 1 to 5;
(Step 2) preparing a plant mixture solution by mixing the transgenic plant obtained in (Step 1) with a protein extraction buffer solution;
(Step 3) recovering a mixed solution from which plant debris is removed from the mixture solution obtained in (Step 2);
(Step 4) injecting the mixture solution obtained in (Step 3) into an agarose-filled column to adsorb a polyhistidine-tagged recombinantAMV capsid protein to the agarose;
(Step 5) washing by injecting a washing solution into the column; and
(Step 6) eluting the recombinant protein adsorbed on agarose by injecting an elution solution into the column.

8. The method of claim 7, wherein the protein extraction buffer solution satisfies at least one of the following features:
(1) comprising 10 to 100 mM Tris, 100 to 1,500 mM magnesium chloride (MgCl₂), 0.01 to 1% Triton X-100 (polyethylene glycol p-(1,1,3,3-tetramethylbutyl)-phenylether), and 5 to 300 mM imidazole; or
(2) pH is 7 to 9.

9. The method of claim 7, wherein the agarose is iminodiacetic acid (Ni-IDA) agarose.

10. The method of claim 7, wherein (Step 1) transforms a plant by using bacteria into which the recombinant vector is introduced.

11. The method of claim 10, wherein the bacteria is *Agrobacterium tumefaciens.*

12. The method of claim 7, wherein the plant is a dicotyledonous plant selected from the group consisting of Arabidopsis, soybean, tobacco, eggplant, pepper, potato, tomato, Chinese cabbage, cabbage and lettuce; or a monocotyledonous plant selected from the group consisting of rice, barley, wheat, rye, corn, sugar cane, oat and onion.

13. A recombinantAMV capsid protein, which is eluted by the method according to claim 7.

14. A method for producing recombinant virus-like particles (VLP), comprising the following steps:
(Step 1) transforming a plant by using the recombinant vector according to any one of claims 1 to 5;
(Step 2) preparing a plant mixture solution by mixing a transgenic plant obtained in (Step 1) with a protein extraction buffer solution;
(Step 3) recovering a mixture solution from which plant debris is removed from the mixture solution obtained in (Step 2);
(Step 4) injecting the mixture solution obtained in (Step 3) into an agarose-filled column to adsorb a polyhistidine-tagged recombinantAMV capsid protein to the agarose;
(Step 5) washing by injecting a washing solution into the column;
(Step 6) injecting an elution solution into the column to elute the recombinant protein adsorbed on the agarose; and
(Step 7) preparing a protein eluted in (Step 6) into virus-like particles.

15. The method of claim 14, wherein (Step 7) prepares virus-like particles by replacing the protein extraction buffer solution including the recombinant AMV capsid protein with a buffer solution for assembling virus-like particles.

16. The method of claim 15, wherein the buffer solution for assembling virus-like particles satisfies at least one of the following features:
(1) comprising 20 to 100 mM sodium pyrophosphate and 100 to 1,000 mM sodium chloride (NaCl); or
(2) pH is 6.9 to 7.5.

17. The method of claim 14, further comprising the step of:
performing size exclusion chromatography to purify self-assembled recombinant AMV virus-like particles.

18. Recombinant AMV virus-like particles, which are recombinant AMV virus-like particles that are produced by the method according to claim 14 and satisfy at least one of the following features:
(1) showing a molecular weight of about 2,000 kDa or more in size-exclusion chromatography;
(2) having a diameter of 10 nm or more to 40 nm or less when observed with a transmission electron microscope after staining by negative staining; or
(3) when observed with a cryogenic transmission electron microscope and reconstructing a 3-dimensional structure, the recombinant AMV virus-like particles are spherical VLPs (3x20) in which a total of 20 trimeric complexes are arranged in a regular arrangement.

19. A method for preparing a vaccine composition comprising recombinantAMV virus-like particles, the method comprising the following steps:
(Step 1) transforming a plant by using the recombinant vector according to any one of claims 1 to 5;
(Step 2) isolating and purifying a recombinant AMV capsid protein from a transgenic plant obtained in (Step 1);
(Step 3) producing virus-like particles from the recombinant AMV capsid protein obtained in (Step 2);
(Step 4) identifying the 3D structure of the virus-like particles obtained in (Step 3) by using a Cryo-electron microscope; and
(Step 5) preparing a vaccine composition comprising the virus-like particles.

20. The method of claim 19, wherein the method for preparing a vaccine composition further comprises the step of adding an adjuvant.

21. The method of claim 19, wherein the method satisfies one of the following features:
(1) the recombinant vector of (Step 1) further comprises a polynucleotide encoding an antigen protein, and the polynucleotide encoding the antigen protein is linked to the N-term end of a polynucleotide encoding an AMV capsid protein; or
(2) (Step 5) comprises the step of conjugating an antigen protein to a surface of the virus-like particles.

22. A vaccine composition, which is prepared by the method according to claim 19.

23. A method for preparing a drug delivery system comprising recombinant AMV virus-like particles, the method comprising the following steps:
(Step 1) transforming a plant by using the recombinant vector according to any one of claims 1 to 5;
(Step 2) isolating and purifying a recombinant AMV capsid protein from a transgenic plant obtained in (Step 1);
(Step 3) preparing a recombinantAMV capsid protein trimer by replacing the recombinant AMV capsid protein solution obtained in (Step 2) with a first buffer solution;
(Step 4) adding a drug to the solution of (Step 3); and
(Step 5) producing virus-like particles by replacing the first buffer solution of (Step 4) with a second buffer solution.

24. The method of claim 23, wherein the first buffer solution and the second buffer solution of (Step 5) satisfy the following features:
(1) the first buffer solution comprises 10 to 100 mMTris and 100 to 500 mM sodium chloride, and has a pH of 6.9 to 7.5; and
(2) the second buffer solution comprise s 20 to 100 mM sodium pyrophosphate and 100 to 1,000 mM sodium chloride, and has a pH of 6.9 to 7.5.
